# EUROPEAN PATENT SPECIFICATION

(11) **EP 1 827 138 B1**
(45) Date of publication and mention of the grant of the patent: **28.04.2010**
(21) Application number: 05814321.5
(22) Date of filing: 12.12.2005
(51) Int. Cl.: A23L 1/30, A23L 2/39, A61P 37/02

(54) **DRY CONCENTRATE FOR MAKING DIETETIC SOFT DRINK CONTAINING Fe, BEE BREAD AND VITAMIN C AND ITS PRODUCTION PROCESS**
TROCKENKONZENTRAT FÜR DIE HERSTELLUNG EINES DIÄTETISCHEN SOFTDRINKS ENTHALTEND FE, BIENENBROT UND VITAMIN C, SOWIE VERFAHREN ZU SEINER HERSTELLUNG
CONCENTRAT SEC POUR LA FABRICATION D'UNE BOISSON NON ALCOOLISÉE DIÉTÉTIQUE CONTENANT Fe, DU PAIN D'ABEILLE ET DE LA VITAMINE C, ET PROCÉDÉ DE FABRICATION DUDIT CONCENTRAT

(30) Priority: 13.12.2004 YU 108004
(43) Date of publication of application: 05.09.2007
(73) Proprietor: Mogorovic, Milos, 11000 Beograd (YU)
(72) Inventor: Mogorovic, Milos, 11000 Beograd (YU)
(74) Representative: Schurack, Eduard F.
(86) International application number: PCT/YU2005/000029
(87) International publication number: WO 2006/066282

(56) References cited:
- US-A- 4 504 516
- US-A1- 2002 058 088
- DATABASE WPI Section Ch, Week 198410 Derwent Publications Ltd., London, GB; Class D16, AN 1984-058402 XP002366395 & JP 59 014783 A (KUTSUZAWA Y) 25 January 1984 (1984-01-25)

## Description

### FIELD OF ART TO WHICH THIS INVENTION APPLIES

The subject of this invention, generally speaking, falls in the category of nonalcoholic drinks and specifically relates to dry concentrate for making a dietetic soft drink containing Fe, beebread and vitamin C and its production process.

### TECHNICAL PROBLEM

The technical problem resolved with this invention consists of the following: how to produce a dietetic soft drink with beebread, Fe and vitamin C that, in addition to its refreshing property, has a strengthening effect on the immune system and raises iron concentration, but such drink must not damage tooth enamel and should be suitable for consumption by children, the elderly, pregnant women, etc.

### STATE OF ART

It is known that around the world and in our country consumption of soft drinks constantly increases and among them leaders are products manufactured by well-known companies like Coca Cola, Pepsi Cola, etc. Consumers largely use those drinks both at home and in restaurants and at other places. Despite powerful advertising used to increase sales of non-alcoholic drinks, one could conclude that needs in this area have been fulfilled. Occasional news about their harmfulness has resulted in the need for new products containing less harmful ingredients (various kinds of sugar, artificial acids and aromas, preserves, etc.). Wishing to create a healthy drink made of natural components known to have specific positive effects on the human body, the author has started to produce a nonalcoholic drink that, apart from its refreshing effect, has a positive stimulating influence especially on the immune system.
Such product is made of natural components whose contents can be maximally declared and recommended from the medical point of view.
When reviewing patent documentation the following patent applications have been found:
- P 791/93 PREPARATION BASED ON HOMEY AND ITS PRODUCTION PROCESS by inventor Novakovic Tomislav of Belgrade;
- P 2401/87, PRODUCTION PROCESS FOR NUTRITIOUS DRINK by inventor Petar Celik of Backa Palanka;

### - P 1720/83 ERGOTONIK - PROTECTIVE SOFT AND NUTRITIOUS DRINK FOR

WORKERS IN HOT PLANTS by inventor Trickovic Kostadin of Nis for which it has been established by comparison that, apart from the fact that they are part of the same classification symbol as the subject invention and that they have associative names, they cannot be compared with the subject invention.
The US 4,504,516 discloses a powdered honey product containing 50-85% of honey or a mixture of honey and at least one bee product selected from the group consisting of beebread, pollen, royal jelly, drone syrup, green syrup, beeswax, propolis and propolis extract, 5-25% silica and 25-35% polymeric carbohydrate.

### FUNDAMENTAL NATURE OF INVENTION

The fundamental nature of this invention is the fact that the following raw materials are used for its production: anhydrous citric acid, MgO or SiO₂ (micronized silicium dioxide), mixture of natural aroma and color, sucrose of which, before all, are those basic ones (lyophilized beebread, vitamin C and chelate iron), natural with tested positive effects that in a well balanced ratio have, apart from refreshing influence, a stimulating effect on the human body and especially on the immune system.
New in this invention is the fact that by using the drink according to the invention chelate iron is taken in the body and due to its insolubility it does not result in the damage of the tooth enamel. It is known that the chemical composition of beebread is very complex and that in its natural form it looses its positive properties within 48 hours. By applying the process of lyophilization beebread is produced in its lyophilized form and thus a broad spectrum of its effects is fully preserved and duration of its positive effects is extended to 2 years. Such use of beebread prevents use of preserves and additives and this improves product quality from the health point of view.
Regarding soft and non-alcoholic drinks known to the author, the subject invention has several advantages over them like the following:
- it can be used by persons of all ages, children, adults, pregnant and nursing women;
- its preventive properties are undisputable as there is an evident immunological effect;
- it does not increase body weight;
- it is produced relatively easily and simply and the process for its production does not require special devices and costly technological equipment:
- it increases iron concentration in the body.

### DETAILED DESCRIPTION OF INVENTION

First raw materials to produce dry concentrate for making the dietetic soft drink are:
- lyophilized beebread
- chelate iron
- vitamin C
- anhydrous citric acid
- ether mixed wild fruit oil
- SiO₂ (micronized silicium dioxide)
- malt dextrin
- anthocyanin
- betaine
- sucrose

*Beebread* (thanks to its healing properties has been widely used in medicine since ancient times), which is the most important ingredient of dry concentrate according to the invention, has a high biological value and contains proteins, vitamins and oligo-elements in superbly balanced quantities that are physiologically acceptable to human body.

*Chelate iron* is easily absorbed from GITA, up to 80% and unlike inorganic salts with resorption up to 25% is has a good property not to damage tooth enamel as inorganic salts and it is especially suitable for use by children pregnant women, etc.

*Vitamin* C (has a protective and bio-stimulating effect) is part of enzyme systems of our body and is necessary for its normal functioning, maintenance and improvement of the immune system.

*Citric acid* serves to produce satisfactory refreshing taste and other powdered fruit acids can be used instead of it, but the commercial effect is best when citric acid is used in this drink.

*Ether mixed wild fruit oil* gives adequate aroma.

*SiO₂* prevents agglomeration of particles and allows easier redispersion and flow. By using SiO₂ ether oil is converted from the liquid to the solid state and such state remains unchanged. It is also possible to use MgO instead of SiO₂ and this is a matter of choice by the manufacturer of dry concentrate according to this invention.
Malt dextrin is also used as a carrier for conveying ether orange oil to the solid state and for adjustment of optimum concentration in the process of pre-preparation of powdered aroma for the production of drink concentrate.
Betaine (extracted from beet) is used as color in this example of the invention.
Anthocyanin (extracted from blueberries or purple grapes) serves to get desired color of dry concentrate.
Sucrose (C₁₂H₂₂O₁₁) is used as a earner and sweetener.
For easier production or due to lack of some raw materials on the market it is possible to use natural aroma or ready natural powdered aroma and color for making the subject dry concentrate and on the market they can be found under different commercial names and are manufactured by companies FRUKT AMINE, ZIVODAN RUR, ETOL, etc. in various concentrations.
First stage in the production of the subject dietetic product is to convey aroma and color to the solid state. Firstly, ether mixed wild fruit oil 5 mass % is coarsely mixed with SiO₂ 10-20 mass % after which betaine is added 20 mass % and anthocyanin 0,2 mass% and supplemented with malt dextrin up to 100 mass %, it is all mixed and sieved through a chromium metal or plastic sieve with openings of 0,75 mm. Coarse mixing of the mentioned components is done during the first stage manually and then in a standard chromium granulator. It is necessary to mention that at this stage during the conveying of aroma and color to the solid state larger quantities of raw materials are used for precise dosing.
The next stage consists of preparation of beebread triturate 1 : 10 so that 10 mass % of liophilized beebread is coarsely mixed with 90 mass % of sucrose in a homogenous mixture that is sieved through a sieve with openings of 0,75 mm. At this stage larger quantities of raw materials are used for precise dosing.
Sucrose is firstly prepared so that it is ground in a standard chromium hammering mill to the granulate of specific fineness and 40% of granulate passes through the sieve with openings of 0,75 mm and the rest through the sieve of openings of 1 mm.
Chelate iron is sieved through a sieve with openings of 0,15-0,30 mm.
Vitamin C is prepared so that it is sieved through a sieve with openings of 0,75 mm. Preparation of anhydrous citric acid is also made with sieving through the sieve with openings of 1 mm.
All substances taking part in the production of this dietetic drink and prepared according to the abovementioned stages are measured precisely in the following mass %:
- chelate iron (20%) 0,425%
- beebread triturate 0,175 %
- vitamin C 0,4 %
- anhydrous citric acid 3,75%
- mixture of aroma and color 4 %
- sucrose 90,25%
- SiO₂ 1%
and in the mentioned order are thrown in the chromium mixer and are mixed until a homogenous mixture is obtained Duration of mixing varies and depends on mixer characteristics. If the mixer is a cube, double cone or the like, mixing lasts maximally 20 minutes. If the mixer has cutting blades and a knife or if it performs against-the-flow double mixing then mixing lasts maximally 5 minutes. Upon completed mixing, concentration of substance samples taken from various parts of the mixer can maximally differ up to:
- beebread 10%
- chelate iron 10%
- vitamin C 10%
- mixture of aroma and color 5%
- anhydrous citric acid 5%
- sucrose up to 0,4%

In order to prove that the invention can be produced, the basic example of its make shows production of dry concentrate with raw materials consisting of ether mixed wild fruit oil, SiO₂ (micronized silicium dioxide), malt dextrin, anthocyanin and betaine , but also, without reducing quality of the drink, prepared natural dry orange aroma can be used with mixed natural colors or already prepared dry natural mixed wild fruit aroma with mixed natural colors.

It is important to mention that the premises where production is carried out of the subject invention, apart from general requirements for the production of dietetic produce intended for human use, must also meet conditions in respect of relative air humidity that must be adjusted depending on outside temperature and it must be below 40% of relative humidity.

Such mixture is packed in suitable packing that apart from bulk function and mechanical protection of produce must provide steam barrier - or rather to prevent moisture reaching dry concentrate.

Dry concentrate of this dietetic drink, according to the invention for making consumable drink, is mixed with water in the best ratio of 60 g to 11 of water.

### METHOD OF INDUSTRIAL OR OTHER APPLICATION OF INVENTION

The subject invention, according to this example can be produced in plants having registration, adequate equipment and staff for the manufacturing of dietetic products.

Subject production is easy to organize based on description given in this invention application.

## Claims

1. Dry concentrate for making a dietetic soft drink IS **CHARACTERIZED BY** THAT it consists of raw materials: lyophilized beebread, chelate iron (20%), vitamin C, powdered fruit acid, especially anhydrous citric acid, micronized SiO₂ or MgO, mixture of natural aroma and color, and sucrose as a carrier and sweetener.

2. The dry concentrate of claim 1 IS **CHARACTERIZED BY** THAT it consists of raw materials measured in the following mass proportions: lyophilized beebread 0,0175 mass%, chelate iron (20%) 0,425 mass%, vitamin C 0,4 mass%, anhydrous citric acid 3,75 mass%, micronized SiO₂ 1,0 mass%, mixture of natural aroma and color 4 mass%, and sucrose 90,4075 mass%.

3. The dry concentrate of claim 1 in which the mixture of natural aroma and color consists of ether mixed wild fruit oil 5 mass%, micronized SiO₂ 10-20 mass%, betaine 20 mass%, anthocyanin 0,2 mass%, supplemented with malt dextrin up to 100 mass% or in which the mixture of natural aroma and color comprises natural dry orange aroma with mixed natural colors or dry natural mixed wild fruit aroma with mixed natural colors or natural aroma or ready natural powdered aroma and color.

4. The dry concentrate according to claim 3 IS **CHARACTERIZED BY** THAT the mixture of natural aroma and color are conveyed to the solid state so that ether mixed wild fruit oil is coarsely mixed with SiO₂ after which anthocyanin and betaine are added and supplemented with malt dextrin and then it is all mixed and sieved through a sieve with openings of 0,75 mm.

5. The dry concentrate according to claim 1 IS **CHARACTERIZED BY** THAT beebread is coarsely mixed with sucrose in a homogenous mixture and then sieved through a sieve with openings of 0,75 mm while sucrose is firstly ground in a standard chromium hammering mill to fine granulate so that 40% passes through the sieve with openings of 0,75 mm and the remainder through the sieve with openings of 1 mm.

6. Process for producing a dry concentrate with beebread for making a dietetic soft drink IS **CHARACTERIZED BY** THAT the substances taking part in the production of dry concentrate in precisely measured mass percentages: beebread triturate 0,175 mass% sieved through a sieve with openings 0,75 mm, the beebread triturate containing 10 mass% of lyophilized beebread and 90 mass% of sucrose, chelate iron (20%) 0,425 mass%, vitamin C 0,4 mass% sieved through a sieve with openings 0,75 mm, mixture of natural aroma and color 4 mass% sieved through a sieve with openings of 0,75 mm, powdered fruit acid, especially anhydrous citric acid, 3,75 mass% sieved through a sieve with openings of 1 mm and suchrose 90,25 mass% sieved through a sieve with openings of 1 mm, micronized SiO₂ or MgO 1 mass% according to the mentioned order are thrown in the chromium mixer and are mixed until a homogenous mixture is obtained.

7. The process of claim 6 in which the mixture of natural aroma and color consists of ether mixed wild fruit oil 5 mass%, micronized SiO₂ 10-20 mass%, betaine 20 mass%, anthocyanin 0,2 mass%, supplemented with malt dextrin up to 100 mass% or in which the mixture of natural aroma and color comprises natural dry orange aroma with mixed natural colors or dry natural mixed wild fruit aroma with mixed natural colors or natural aroma or ready natural powdered aroma and color.

8. The process of claim 7 in which the mixture of natural aroma and color are conveyed to the solid state so that ether mixed wild fruit oil is coarsely mixed with SiO₂ after which anthocyanin and betaine are added and supplemented with malt dextrin and then it is all mixed and sieved through a sieve with openings of 0,75 mm.

9. The process of claim 6 in which beebread is coarsely mixed with sucrose in a homogenous mixture and then sieved through a sieve with openings of 0,75 mm while sucrose is firstly ground in a standard chromium hammering mill to fine granulate so that 40% passes through the sieve with openings of 0,75 mm and the remainder through the sieve with openings of 1 mm.

10. The process of claim 6 in which the chelate iron is sieved through a sieve with openings of 0,15-0,30 mm.

11. Dietetic drink which is made by mixing the dry concentrate of claims 1 to 5 or the dry concentrate produced according to one of claims 6 to 10 before consumption with water in the best ratio of 60 g to 1 1 of water.

## Patentansprüche

1. Trockenkonzentrat zur Herstellung eines diätetischen Softdrinks, **dadurch gekennzeichnet, dass** es aus Rohmaterialien besteht: lyophilisiertem Bienenbrot, Chelateisen (20 %), Vitamin C, pulverisierter Fruchtsäure, insbesondere wasserfreier Zitronensäure, mikronisiertem SiO₂ oder MgO, einem Gemisch aus natürlichem Aroma und Farbe und Saccharose als Träger und Süßungsmittel.

2. Trockenkonzentrat nach Anspruch 1, **dadurch gekennzeichnet, dass** es aus Rohmaterialien besteht, die in den folgenden Massenanteilen abgemessen sind: lyophilisiertes Bienenbrot 0,0175 Massen-%, Chelateisen (20 %) 0,425 Massen-%, Vitamin C 0,4 Massen-%, wasserfreie Zitronensäure 3,75 Massen-%, mikronisiertes SiO₂ 1,0 Massen-%, ein Gemisch aus natürlichem Aroma und Farbe 4 Massen-% und Saccharose 90,4075 Massen-%.

3. Trockenkonzentrat nach Anspruch 1, wobei das Gemisch aus natürlichem Aroma und Farbe aus mit Ether vermischtem Wildfruchtöl, 5 Massen-%, mikronisiertem SiO₂ 10-20 Massen-%, Betain, 20 Massen-%, Anthocyanin, 0,2 Massen-%, ergänzt mit Malzdextrin bis zu 100 Massen-%, besteht, oder wobei das Gemisch aus natürlichem Aroma und Farbe natürliches Trockenorangenaroma mit gemischten natürlichen Farben oder trockenes natürliches gemischtes Wildfruchtaroma mit gemischten natürlichen Farben oder natürlichem Aroma oder fertiges natürliches pulverisiertes Aroma und Farbe umfasst.

4. Trockenkonzentrat nach Anspruch 3, **dadurch gekennzeichnet, dass** das Gemisch aus natürlichem Aroma und Farbe in den festen Zustand überführt wird, so dass mit Ether gemischtes Wildfruchtöl mit SiO₂ grob vermischt wird, wonach Anthocyanin und Betain zugegeben werden und mit Malzdextrin ergänzt werden, und dann alles vermischt und durch ein Sieb mit Öffnungen von 0,75 mm gesiebt wird.

5. Trockenkonzentrat nach Anspruch 1, **dadurch gekennzeichnet, dass** Bienenbrot mit Saccharose in einem homogenen Gemisch grob vermischt und dann durch ein Sieb mit Öffnungen von 0,75 mm gesiebt wird, während Saccharose zuerst in einer Standard-Chromhammermühle zu einem feinen Granulat vermahlen wird, so dass 40 % durch das Sieb mit Öffnungen von 0,75 mm und der Rest durch das Sieb mit Öffnungen von 1 mm hindurchgeht.

6. Verfahren zur Herstellung eines Trockenkonzentrats mit Bienenbrot zur Herstellung eines diätetischen Softdrinks, **dadurch gekennzeichnet, dass** die Substanzen, die an der Herstellung des Trockenkonzentrats teilnehmen, in präzise abgemessenen Massenprozentsätzen: Bienenbrottriturat, 0,175 Massen-%, das durch ein Sieb mit Öffnungen von 0,75 mm gesiebt wird, wobei das Bienenbrottriturat 10 Massen-% lyophilisiertes Bienenbrot und 90 Massen-% Saccharose enthält, Chelateisen (20 %), 0,425 Massen-%, Vitamin C, 0,4 Massen-%, das durch ein Sieb mit Öffnungen von 0,75 mm gesiebt wird, ein Gemisch von natürlichem Aroma und Farbe, 4 Massen-%, das durch ein Sieb mit Öffnungen von 0,75 mm gesiebt wird, pulverisierte Fruchtsäure, insbesondere wasserfreie Zitronensäure, 3,75 Massen-%, die durch ein Sieb mit Öffnungen von 1 mm gesiebt wird, und Saccharose, 90,25 Massen-%, die durch ein Sieb mit Öffnungen von 1 mm gesiebt wird, mikronisiertes SiO₂ oder MgO, 1 Massen-%, gemäß der erwähnten Reihenfolge in den Chrommischer geschüttet werden und gemischt werden, bis ein homogenes Gemisch erhalten wird.

7. Verfahren nach Anspruch 6, wobei das Gemisch aus natürlichem Aroma und Farbe aus mit Ether gemischtem Wildfruchtöl, 5 Massen-%, mikronisiertem SiO₂, 10-20 Massen-%, Betain, 20-Massen-%, Anthocyanin, 0,2 Massen-%, ergänzt mit Malzdextrin bis zu 100 Massen-%, besteht oder wobei das Gemisch aus natürlichem Aroma und Farbe natürliches Trockenorangenaroma mit gemischten natürlichen Farben oder trockenes natürliches gemischtes Wildfruchtaroma mit gemischten natürlichen Farben oder natürlichem Aroma oder fertiges natürliches pulverisiertes Aroma und Farbe umfasst.

8. Verfahren nach Anspruch 7, wobei das Gemisch aus natürlichem Aroma und Farbe in den festen Zustand überführt wird, so dass mit Ether vermischtes Wildfruchtöl mit SiO₂ grob vermischt wird, wonach Anthocyanin und Betain zugegeben werden und mit Malzdextrin ergänzt werden, und dann alles vermischt und durch ein Sieb mit Öffnungen von 0,75 mm gesiebt wird.

9. Verfahren nach Anspruch 6, wobei Bienenbrot mit Saccharose in einem homogenen Gemisch grob vermischt wird und dann durch ein Sieb mit Öffnungen von 0,75 mm gesiebt wird, während Saccharose zuerst in einer Standard-Chromhammermühle zu feinem Granulat vermahlen wird, so dass 40 % durch das Sieb mit Öffnungen von 0,75 mm und der Rest durch das Sieb mit Öffnungen von 1 mm hindurchgeht.

10. Verfahren nach Anspruch 6, wobei das Chelateisen durch ein Sieb mit Öffnungen von 0,15-0,30 mm gesiebt wird.

11. Diätgetränk, das durch Mischen des Trockenkonzentrats nach den Ansprüchen 1 bis 5 oder des nach einem der Ansprüche 6 bis 10 hergestellten Trockenkonzentrats vor dem Verzehr mit Wasser im besten Verhältnis von 60 g zu 1 I Wasser hergestellt wird.

## Revendications

1. Concentré sec destiné à réaliser une boisson gazeuse diététique, **caractérisée en ce qu'**il se compose des matières premières suivantes : du pain d'abeille lyophilisé, du fer chélaté (20 %), de la vitamine C, de l'acide de fruit réduit en poudre, en particulier de l'acide citrique anhydre, du SiO₂ ou du MgO micronisé, un mélange d'arôme naturel et de colorant et du sucrose en tant que support et qu'édulcorant.

2. Concentré sec selon la revendication 1, **caractérisé en ce qu'**il se compose des matières premières, mesurées dans les proportions en masses suivantes : pain d'abeille lyophilisé : 0,0175 % en masse, fer chélaté (20 %) : 0,425 % en masse, vitamine C : 0,4 % en masse, acide citrique anhydre : 3,75 % en masse, SiO₂ micronisé : 1,0 % en masse, mélange d'arôme naturel et de colorant : 4 % en masse et sucrose : 90,4075 % en masse.

3. Concentré sec selon la revendication 1, dans lequel le mélange d'arôme naturel et de colorant consiste en de l'huile de fruit sauvage, mélangée avec de l'éther : 5 % en masse, du SiO₂ micronisé : 10 - 20 % en masse, de la bétaïne : 20 % en masse, de l'anthocyane : 0,2 % en masse, supplémenté avec de la dextrine de malt jusqu'à 100 % en masse ou dans lequel le mélange d'arôme naturel et de colorant comprend de l'arôme d'orange sec naturel avec des colorants naturels mélangés ou de l'arôme de fruit sauvage mélangé naturel sec avec des colorants naturels mélangés ou de l'arôme naturel ou de l'arôme réduit en poudre naturel prêt et du colorant.

4. Concentré sec selon la revendication 3, **caractérisé en ce que** le mélange d'arôme naturel et de colorant est amené à l'état solide, de sorte que de l'huile de fruit sauvage, mélangée avec de l'éther, est mélangée grossièrement avec du SiO₂, opération après laquelle l'anthocyane et la bétaïne sont ajoutées et supplémentées avec de la dextrine de malt ; puis le tout est mélangé et passé à travers un tamis, doté d'ouvertures de 0,75 mm.

5. Concentré sec selon la revendication 1, **caractérisé en ce que** le pain d'abeille est mélangé grossièrement avec du sucrose en un mélange homogène puis passé à travers un tamis, doté d'ouvertures de 0,75 mm, tandis que le sucrose est tout d'abord broyé dans un broyeur à marteaux revêtu de chrome standard pour obtenir un granulé fin, de sorte que 40 % passent à travers le tamis, doté d'ouvertures de 0,75 mm et le reste à travers le tamis, doté d'ouvertures d'1 mm.

6. Procédé destiné à produire un concentré sec avec du pain d'abeille pour réaliser une boisson gazeuse diététique, **caractérisé en ce que** les substances, qui prennent part à la production de concentré sec dans des pourcentages en masse, mesurés avec précision : du triturat de pain d'abeille : 0,175 % en masse, passé à travers un tamis, doté d'ouvertures de 0,75 mm, le triturat de pain d'abeille contenant 10 % en masse de pain d'abeille lyophilisé et 90 % en masse de sucrose, du fer chélaté (20 %) : 0,425 % en masse, de la vitamine C : 0,4 % en masse, passée à travers un tamis, doté d'ouvertures de 0,75 mm, un mélange d'arôme naturel et de colorant : 4 % en masse, passé à travers un tamis, doté d'ouvertures de 0,75 mm, de l'acide de fruit réduit en poudre, en particulier de l'acide citrique anhydre : 3,75 % en masse, passé à travers un tamis, doté d'ouvertures d'1 mm et du sucrose : 90,25 % en masse, passé à travers un tamis, doté d'ouvertures d'1 mm, du SiO₂ ou du MgO micronisé : 1 % en masse, suivant l'ordre mentionné, sont projetées dans le mélangeur revêtu de chrome et sont mélangées jusqu'à ce qu'un mélange homogène soit obtenu.

7. Processus selon la revendication 6, dans lequel le mélange d'arôme naturel et de colorant se compose d'huile de fruit sauvage, mélangée avec de l'éther : 5 % en masse, de SiO₂ micronisé : 10 - 20 % en masse, de la bétaïne : 20 % en masse, de l'anthocyane : 0,2 % en masse, supplémenté avec de la dextrine de malt jusqu'à 100 % en masse ou dans lequel le mélange d'arôme naturel et de colorant comprend de l'arôme d'orange sec naturel avec des colorants naturels mélangés ou de l'arôme de fruit sauvage mélangé naturel sec avec des colorants naturels mélangés ou de l'arôme naturel ou de l'arôme réduit en poudre naturel prêt et du colorant.

8. Processus selon la revendication 7, dans lequel le mélange d'arôme naturel et de colorant est amené à l'état solide, de sorte que de l'huile de fruit sauvage, mélangée avec de l'éther, est mélangée grossièrement avec du SiO₂ opération après laquelle de l'anthocyane et de la bétaïne sont ajoutées et supplémentées avec de la dextrine de malt puis le tout est mélangé et passé à travers un tamis, doté d'ouvertures de 0,75 mm.

9. Processus selon la revendication 6, dans lequel du pain d'abeille est mélangé grossièrement avec du sucrose en un mélange homogène puis passé à travers un tamis, doté d'ouvertures de 0,75 mm, tandis que du sucrose est tout d'abord broyé dans un broyeur à marteaux revêtu de chrome standard pour en faire des granulés fins, de sorte que 40 % passent à travers le tamis, doté d'ouvertures de 0,75 mm et le reste à travers le tamis, doté d'ouvertures de 1 mm.

10. Processus selon la revendication 6, dans lequel le fer chélaté est passé à travers un tamis, doté d'ouvertures de 0,15 - 0,30 mm.

11. Boisson diététique, faite en mélangeant le concentré sec des revendications 1 à 5 ou le concentré sec, produit selon l'une des revendications 6 à 10, avant sa consommation avec de l'eau dans la meilleure proportion de 60 g dans 1 l d'eau.
